(19) Europäisches Patentamt
European Patent Office
Office européen des brevets

(11) Veröffentlichungsnummer: **0 201 738**
**A1**

# (12) EUROPÄISCHE PATENTANMELDUNG

(21) Anmeldenummer: 86105154.8

(22) Anmeldetag: 15.04.86

(51) Int. Cl.[4]: C 07 C 19/02
C 07 C 17/38

(30) Priorität: 16.04.85 DE 3513522

(43) Veröffentlichungstag der Anmeldung:
20.11.86 Patentblatt 86/47

(84) Benannte Vertragsstaaten:
BE CH DE FR GB IT LI NL

(71) Anmelder: WACKER-CHEMIE GMBH
Prinzregentenstrasse 22
D-8000 München 22(DE)

(72) Erfinder: Schneider, Otto, Dr., Dipl.-Chem.
Stegerwaldstrasse 5
D-8263 Burghausen(DE)

(72) Erfinder: Müller, Reinhardt, Dr., Dipl.-Chem.
Karl-Gross-Strasse 12
D-8263 Burghausen(DE)

(72) Erfinder: Weissenbach, Georg, Dipl.-Ing.
Bachstrasse 14
D-8263 Burghausen(DE)

## (54) Verfahren zur Reinigung von Monohalogenalkanen.

(57) Monohalogenalkane, die mindestens Dialkylether als Verunreinigung enthalten, werden dadurch gereinigt, daß die durch Adsorptionsmittel, die aus Oxyden des Siliciums und/oder Aluminiums sowie gegebenenfalls weiterer Metallatome aufgebaut und frei von Halogeniden des Phosphors sind, bei – 30°C bis 50°C geleitet werden.



Croydon Printing Company Ltd.

W A C K E R - C H E M I E
G M B H

München, den 6.3.1985
Dr. Ru/rei

0201738



Wa 8505-S

## Verfahren zur Reinigung von Monohalogenalkanen

Verfahren zur Reinigung von Monohalogenalkanen, die mindestens Dialkylether als Verunreinigung enthalten, sind bereits bekannt. Hierzu wird auf z.B. Chemical Abstracts, Vol. 90 (1979), 54450y verwiesen.

Es bestand nun die Aufgabe, ein Verfahren zur Reinigung von Monohalogenalkanen, die mindestens Dialkylether als Verunreinigung enthalten, bereitzustellen, das mit verhältnismäßig geringem Aufwand für die für seine Durchführung erforderliche Vorrichtung und Energie sowie die bei dem Verfahren verwendeten Stoffe und die Entsorgung ohne Verlust wertvoller organischer Verbindungen genügend reine Monohalogenalkane liefert und umweltfreundlich ist. Diese Aufgabe wird durch die Erfindung gelöst.

Gegenstand der Erfindung ist ein Verfahren zur Reinigung von Monohalogenalkanen, die mindestens Dialkylether als Verunreinigung enthalten, durch Leiten des zu reinigenden Halogenalkans durch Adsorptionsmittel, die aus Oxyden des Siliciums oder Aluminiums oder des Siliciums und Aluminiums sowie gegebenenfalls weiterer Metallatome aufgebaut sind, bei -30°C bis +50°C, dadurch gekennzeichnet, daß das Adsorptionsmittel frei von Halogeniden des Phosphors ist.

Die bei dem erfindungsgemäßen Verfahren eingesetzten Monohalogenalkane sind vorzugsweise solche der Formel

$$RX ,$$

wobei R ein Alkylrest mit 1 bis 5 Kohlenstoffatomen und X

Fluor, Chlor, Brom oder Jod ist.

Beispiele für Alkylreste R sind der Methyl-, Ethyl-, n-Propyl-, Isopropyl-, n-Butyl-, sec.-Butyl- und tert.-Butylrest, sowie die verschiedenen isomeren Pentylreste.

Als Halogenatom ist Chlor bevorzugt.

Beispielsweise weil es im großen Umfang zur Herstellung von Organosiliciumverbindungen eingesetzt und auch im großen Umfang bei der Umsetzung von Organochlorsilanen mit Methanol erzeugt wird, ist Methylchlorid als zu reinigendes Monohalogenalkan besonders bevorzugt.

Wenn das Methylchlorid durch Umsetzung von Methanol mit Chlorwasserstoff oder Chlorsilanen hergestellt wurde, enthält es als Verunreinigungen Dimethylether, Wasser und Spuren von Methanol. Diese Verunreinigungen werden durch das erfindungsgemäße Verfahren aus dem Monohalogenalkan entfernt. Je weniger Wasser das bei dem erfindungsgemäßen Verfahren eingesetzte Monohalogenalkan enthält, desto später muß das dabei verwendete Adsorptionsmittel regeneriert werden.

Vorzugsweise wird bei dem erfindungsgemäßen Verfahren das zu reinigende Monohalogenalkan in Gasform eingesetzt. Es kann aber auch in flüssiger Form eingesetzt werden.

Vorzugsweise wird das zu reinigende Monohalogenalkan bei 0°C bis 40°C, insbesondere 0°C bis 20°C durch das Adsorptionsmittel geführt.

Beispiele für Adsorptionsmittel, die aus Oxyden des Siliciums und/oder Aluminiums sowie gegebenenfalls weiterer Metallatomen aufgebaut sind, sind Kieselgel (silica gel), poröses Glas, Aktives Aluminiumoxyd in Form von Kugeln oder Strangpreßlingen, synthetische und in der Natur vorkommende Aluminiumsilikate, wie

Bentonite, Montmorillonite und Kaolinit, und Molekularsiebe, insbesondere Zeolithe mit einem Porendurchmesser von mindestens 0,4 Nanometer. Wegen der besonders hohen Aufnahmefähigkeit für die Verunreinigungen von Monohalogenalkanen und verhältnismäßig geringem Energiebedarf für die Regenerierung ist Kieselgel als Adsorptionsmittel bei dem erfindungsgemäßen Verfahren besonders bevorzugt.

Vorzugsweise sind die bei dem erfindungsgemäßen Verfahren eingesetzten Adsorptionsmittel nicht nur frei von Halogeniden des Phosphors, sondern auch frei von anderen Fremdstoffen als Halogeniden des Phosphors, Monohalogenalkan und Verunreinigungen des Monohalogenalkans.

Vorzugsweise wird das erfindungsgemäße Verfahren beim Druck der umgebenden Atmosphäre, also bei 1020 hPa (abs.) oder etwa 1020 hPa (abs.), bis 8000 hPa (abs.) durchgeführt. Niedrigere oder höhere Drücke können angewendet werden, bringen aber keine Vorteile gegenüber dem vorstehend angegebenen Bereich von Drücken.

Nach bzw. während der Durchführung des erfindungsgemäßen Verfahrens kann Adsorptionsmittel, dessen Aufnahmefähigkeit für Verunreinigungen des zu reinigenden Monohalogenalkans nicht mehr genügend groß ist, regeneriert werden. Dieses Regenerieren erfolgt vorzugsweise durch fraktionierte Desorption, beispielsweise durch fraktionierte thermische Desorption. Bei der fraktionierten thermischen Desorption von Adsorptionsmittel, das bei dem erfindungsgemäßen Verfahren zur Reinigung von Methylchlorid eingesetzt wurde, wird während des Erwärmens das bei diesem Verfahren eingesetzten Adsorptionsmittels, dessen Aufnahmefähigkeit für Verunreinigungen des zu reinigenden Monohalogenalkans nicht mehr genügend groß ist, zunächst Methylchlorid frei, das wieder in das erfindungsgemäße Verfahren zurückgeführt werden kann, dann Dimethylether, der z.B. wieder in die Umsetzung von Methanol mit Chlorwasserstoff oder Dimethyldichlorsilan zurückgeführt werden kann, und schließlich Methanol und Wasser frei, die von einander durch Destillation

getrennt oder als Gemisch beispielsweise in die Umsetzung von Alkanol mit Chlorwasserstoff oder Dimethyldichlorsilan zurückgeführt werden können.

Während in einem Bett, das Adsorptionsmittel regeneriert wird, kann mit einem Bett, das Adsorptionsmittel mit genügend grosser Adsorptionsfähigkeit für Verunreinigungen des zu reinigenden Monohalogenalkans enthält, das erfindungsgemäße Verfahren durchgeführt werden.

Anstelle von thermischer Desorption können z.B. auch Verdrängungsdesorption oder Druckwechseldesorption angewandt werden.

Beispiele

Je 200 g der in der folgenden Tabelle angegebenen Adsorptionsmittel werden in ein senkrecht angeordnetes Glasrohr mit einem Innendurchmesser von 55 mm eingefüllt. Das Glasrohr ist von einem Mantel umgeben, durch den Wasser mit der Temperatur von +10°C geleitet wird. Durch das Adsorptionsmittel wird bei 1530 hPa Methylchlorid, das mit Wasserdampf gesättigt ist, die in der folgenden Tabelle angegebenen Mengen Dimethylether enthält und schon vor dem Eintritt in das Adsorptionsmittel die Temperatur von +10°C hat, mit einer Geschwindigkeit von 100 l [gemessen bei 20°C und 960 hPa (abs.)] je Stunde geleitet.

Beim Vergleich der Gewichtsmenge von eingesetztem Adsorptionsmittel mit der Gewichtsmenge von regeneriertem Adsorptionsmittel ergibt sich, daß keine nicht-desorbierbaren Stoffe auf dem Adsorptionsmittel abgelagert wurden.

TABELLE

| Beispiel | Adsorptionsmittel | Schütthöhe mm | $(CH_3)_2O$ +) Gew.-% | Durchsatz ++) $CH_3Cl + (CH_3)_2O$ g | $H_2O$ +++) mg/kg $CH_3Cl$ | $(CH_3)_2O$ adsorbiert je g Adsorptionsmittel g |
|---|---|---|---|---|---|---|
| 1 (I.) | Kieselgel, Perlen ⌀ 2 bis 5 mm | 105 | 2,9 | 350 | 4 | 0,051 |
| 2 (I.) | Kieselgel, gebrochen ⌀ 2 bis 5 mm | 105 | 2,5 | 440 | 2 | 0,055 |
| 3 (I.) | Kieselgel, Perlen ⌀ 2 bis 5 mm | 105 | 0,7 | 1110 | 6 | 0,039 |
| 4 (I.) (II.) | Kieselgel, Perlen ⌀ 2 bis 5 mm | 105 | 2,5 | 600 | 2 | 0,075 |
| 5 (III.)(IV.) | Molekularsieb Stränge, ⌀ 1,5 mm | 110 | 2,7 | 330 | 3 | 0,045 |
| 6 (I.) | $Al_2O_3$, Stränge ⌀ 3 mm | 150 | 2,7 | 120 | 7 | 0,016 |
| 7 (I.) | Montmorillonit | 95 | 3,1 | 50 | 5 | 0,008 |

Fußnoten zur Tabelle

(I.) Vor der Verwendung 16 Stunden im Trockenschrank auf 200°C erwärmt.

(II.) Fast wasserfreies Gemisch aus Methylchlorid und Dimethylether.

(III.) Vor der Verwendung 3 Stunden bei 1 hPa (absolut) auf 300°C erwärmt.

(IV.) Molekularsieb Union Carbide 13X, mit der Zusammensetzung $Na_{86}[(AlO_2)_{86}-(SiO_2)_{106}]\cdot nH_2O$ mit einer Porengröße von 1 Nanometer.

+) Menge des Dimethylethers im eingesetzten Methylchlorid, bezogen auf das Gewicht von Dimethylether und Methylchlorid.

++) Menge, die nach dem Austreten aus dem Adsorptionsmittel höchstens 0,01 Gewichtsprozent Dimethylether, bezogen auf das Gewicht des Methylchlorids, enthält.

+++) Wassergehalt des gereinigten Guts

PATENTANSPRÜCHE:

1. Verfahren zur Reinigung von Monohalogenalkanen, die mindestens Dialkylether als Verunreinigung enthalten, durch Leiten des zu reinigenden Halogenalkans durch Adsorptionsmittel, die aus Oxyden des Siliciums oder Aluminiums oder des Siliciums und Aluminiums sowie gegebenenfalls weiterer Metallatome aufgebaut sind, bei -30°C bis +50°C, dadurch gekennzeichnet, daß das Adsorptionsmittel frei von Halogeniden des Phosphors ist.

2. Verfahren nach Anspruch 1, dadurch gekennzeichnet, daß das Adsorptionsmittel auch frei von anderen Fremdstoffen als Monohalogenalkanen und Verunreinigungen des Monohalogenalkans ist.

3. Verfahren nach Anspruch 1 und 2, dadurch gekennzeichnet, daß als Adsorptionsmittel Kieselgel verwendet wird.

0201738

Europäisches Patentamt

# EUROPÄISCHER RECHERCHENBERICHT

Nummer der Anmeldung

EP 86 10 5154

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | US-A-2 458 819 (H.L. YOWELL et al.)<br>* Patentansprüche *<br>----- | 1-3 | C 07 C 19/02<br>C 07 C 17/38 |
| | | | RECHERCHIERTE SACHGEBIETE (Int. Cl.4) |
| | | | C 07 C 17/00 |

Der vorliegende Recherchenbericht wurde für alle Patentansprüche erstellt.

| Recherchenort | Abschlußdatum der Recherche | Prüfer |
|---|---|---|
| DEN HAAG | 11-08-1986 | VAN GEYT J.J.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82